(19) Europäisches Patentamt
European Patent Office
Office européen des brevets

(11) Publication number: **0 381 533 A2**

(12) **EUROPEAN PATENT APPLICATION**

(21) Application number: 90301146.8

(51) Int. Cl.5: C07F 9/6564, A61K 31/675

(22) Date of filing: 02.02.90

(30) Priority: 02.02.89 US 305684

(43) Date of publication of application: 08.08.90 Bulletin 90/32

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Tolman, Richard L.
29 Upper Warren Way
Warren, New Jersey 07060(US)
Inventor: Maccoss, Malcolm
48 Rose Court
Freehold, New Jersey 07728(US)

(74) Representative: Hesketh, Alan, Dr. et al
European Patent Department Merck & Co., Inc. Terlings Park Eastwick Road
Harlow Essex, CM20 2QR(GB)

(54) **Cyclic monophosphates of purine and pyrimidine acyclonucleosides.**

(57) A compound having the formula B-$CH_2$-X-Z wherein B is a pyrimidine, a substituted pyrimidine, a purine, a substitued purine wherein the substituents on the purine or pyrimidine are selected from amino, hydroxyl halogen, thio or alkylthiol wherein the alkyl moiety of the alkylthiol has 1 to 6 carbons and the purine or substituted purine is not guanine; X is oxygen, sulfur or methylene; Z is

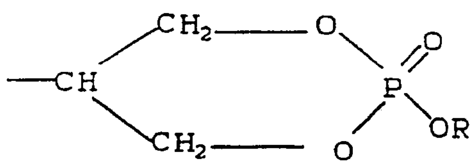

OR

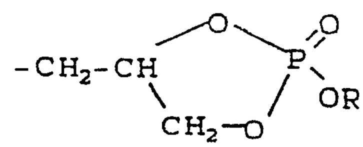

and R is hydrogen or a pharmaceutically acceptable cation.

These compounds have antiviral activity and are effective against the herpes class of viruses such as herpes simplex and cytomegalovirus.

EP 0 381 533 A2

# CYCLIC MONOPHOSPHATES OF PURINE AND PYRIMIDINE ACYCLONUCLEOSIDES AS ANTI-RETROVIRAL AGENTS

## BACKGROUND OF THE INVENTION

The present invention is concerned with novel cyclic monophosphate purine and pyrimidine acyclonuclosides. These compounds are antiviral agents and are effective against the herpes class of viruses such as herpes simplex virus and cytomegalovirus.

The herpes simplex virus (HSV) is a recurrent viral infection characterized by the appearance on the skin or mucous membranes of single or multiple clusters of small vesicles, filled with clear fluid or slightly raised inflammatory bases. HSV has two strains, HSV-1 and HSV-2. HSV-1 commonly causes herpes labialis and keratitis; HSV-2 is usually genital and is transmitted primarily by direct contact with lesions, most often venereally. Presumably, the virus remains dormant in the skin or nerve ganglia, and recurrent herpetic eruptions can be precipitated by overexposure to sunlight, febrile illnesses, physical or emotional stress, or certain foods or drugs. The trigger mechanism is often unknown.

Cytomegalovirus is a subgroup of agents within the herpes group of viruses. Cytomegalovirus infections can occur at any age and can range in severity from a silent infection with no consequences, through diseases manifested by fever, hepatitis, pneumonitis, to stillbirth or perinatal death.

## DESCRIPTION OF THE PRIOR ART

It has been known in the art to use certain purine and pyrimidine acyclonucleosides as antiviral agents. United States Patent No. 4,670,424 discloses cyclic pyrophosphate and bis monophosphate derivatives of purine and pyrimidine acyclonucleosides. European Patent Application No. 82401571.3 discloses cyclic monophosphate guanine derivatives and their use as antiviral agents. Cyclic monophosphate guanine derivatives and their use as antiviral agents is also disclosed in United States Patent No. 4,579,849. However, it has not been known heretofore to use cyclic monophosphate derivatives of acyclonucleosides other than guanine as antiviral agents.

## DESCRIPTION OF THE INVENTION

The present invention is concerned with novel cyclic monophosphates of purine and pyrimidine acyclonuclosides, processes for their preparation, pharmaceutical formulations comprising the novel compounds as active ingredients and methods of treating viral infections with the novel compounds or their pharmaceutical formulations.

The present invention is concerned with novel antiviral compounds of the formula:

$B-CH_2-X-Z$

wherein:

B is a pyrimidine, a substituted pyrimidine, a purine or substituted purine wherein the substituents on the purine or pyrimidine are selected from amino, hydroxyl, halogen, thio or alkylthiol wherein the alkyl moiety of the alkylthiol has 1 to 6 carbon atoms; and the purine or substituted purine is not guanine;

X is oxygen, sulfur or methylene;

Z is

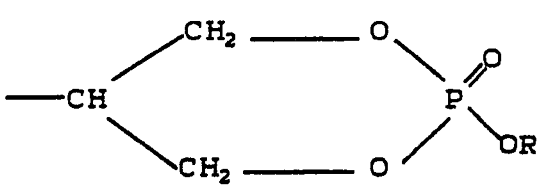

OR

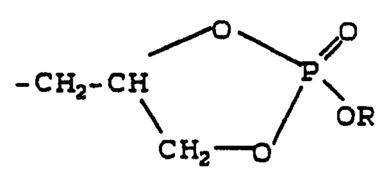

R is hydrogen or a pharmaceutically acceptable cation.

Purines, substituted purines, pyrimidines and substituted pyrimidines are defined as the common heterocylic bases of nucleic acids and include, but are not limited to adenine, cytosine, thymine and uracil.

While purines and pyrimidines that are represented by B, as defined above, may be attached to the $-CB_2$-X-Z moieties from various positions on the purines and pyrimidines, the preferred position for the purines is the 9-position and the preferred position for the pyrimidines is the 1-position.

The term "pharmaceutically acceptable cation" refers to those cations which possess the biological effectiveness and properties of the free compound and which are not biologically or otherwise undesirable. Examples of these cations include ions of sodium, potassium, lithium, calcium, magnesium, ammonium, as well as any other acceptable cation known in the art.

The preferred compound of the present invention is where B is cytosine, X is oxygen, Z is a dioxophosphorinan ring and R is sodium. This compound is represented by the formula:

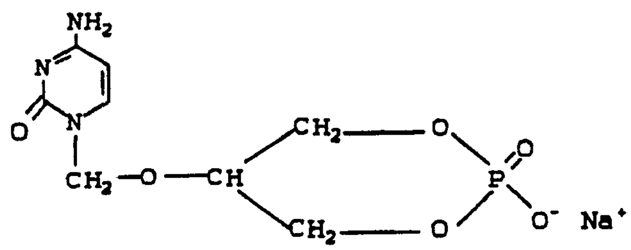

Representative compounds of the present invention include the following:

1-[(1,3-dihydroxy-2-propoxy)methyl] cytosine cyclic monophosphate;
9-[(1,3-dihydroxy-2-propoxy)methyl] adenine cyclic monophosphate;
1-[(1,3-dihydroxy-2-propoxy)methyl] thymine cyclic monophosphate;
1-[(1,3-dihydroxy-2-propoxy)methyl] uracil cyclic monophosphate;
1-[(2,3-dihydroxy-1-propoxy)methyl] cytosine cyclic monophosphate;
9-[(2,3-dihydroxy-1-propoxy)methyl] adenine cyclic monophosphate;
1-[(2,3-dihydroxy-1-propoxy)methyl] thymine cyclic monophosphate;
1-[(2,3-dihydroxy-1-propoxy)methyl] uracil cyclic monophosphate;
1-[(1,3-dihydroxy-2-propoxy)methyl]-5-fluorouracil cyclic monophosphate;
1-[(2,3-dihydroxy-1-propoxy)methyl]-5-fluorouracil cyclic monophosphate;
9-[(1,3-dihydroxy-2-propoxy)methyl]-2,6-diaminopurine cyclic monophosphate;
9-[(2,3-dihydroxy-1-propoxy)methyl]-2-amino-6-methylmercaptopurine cyclic monophosphate.

The most preferred compound of the present invention is sodium 1-[(1,3-dihydroxy-2-propoxy)methyl] cytosine cyclic monophosphate which can also be named as sodium 1-[(2-hydroxy-1,3,2-dioxophosphorinan-5-yl)-oxymethyl]cytosine P-oxide.

Acyclonucleoside cyclic monophosphates of the present invention are prepared by treating the appropriately substituted and protected or unprotected purine or pyrimidine acyclonucleoside with an excess of a phosphorylating agent such as phosphorous oxychloride or pyrophosphoryl chloride in an inert solvent such as triethyl phosphate and optionally, in the presence of a complexing agent such as $SnCl_4$, to solubilize the reactants. The desired product of this reaction, a cyclic monophosphate, is separated from other products such as the bis-monophosphate, by, for instance, gradient elution from a column of anionic exchange resin.

Halopurines which are used as starting materials in the preparation of compounds of the present invention are known in the literature. Halopurine acyclonucleosides are prepared by methods disclosed in European Patent Application 82401571.3, Publication No. 0 074306, published Mar. 16, 1983;

K.K. Ogilvie and M.F. Gillen, Tetrahedron Letters, Vol. 21, 327-330 (1980); and H.J. Shaeffer et al., Nature, Vol. 272, 583-585 (1978). The appropriate halopurine acyclonucleosides can be used directly to make the cyclic monophosphates.

Pyrimidine acyclonucleosides with the desired ring substitution are synthesized by literature methods such as disclosed in European Patent Application 81106460.9, Publication No. 0 046 307, published Feb. 24, 1982; H.M. Abrams et al., J. Heterocyclic Chem., 18, 947-951 (1981); and K.K. Ogilvie and M.F. Gillen, Tetrahedron Letters, Vol. 21,327-330 (1980). The pyrimidine acyclonucleosides are phosphorylated by methods disclosed herein and the ring closed to yield cyclic monophosphates.

In connection with the use of the compounds of this invention for the treatment of viral infections, it is to be noted that they may be administered either alone or in combination with pharmaceutically acceptable

carriers and that such administration can be carried out in both single and multiple dosages. More particularly, a compound of the present invention is administered in an effective unit dosage form.

As used herein the term "effective unit dosage" or "effective unit dose" is denoted to mean a predetermined antiviral amount sufficient to be effective against the virus in vivo. Pharmaceutically acceptable carriers are materials useful for the purpose of administering the medicament, and may be solid, liquid or gaseous materials, which are otherwise inert and medically acceptable and are compatible with active ingredients.

These pharmaceutical compositions may be given parenterally, orally, used as a suppository or pessary, applied topically as an ointment, cream, aerosol, powder, or given as eye or nose drops, etc., depending on whether the preparation is used to treat internal or external viral infections.

For infections the compositions are administered orally or parenterally at dose levels of about 0.1 to 500 mg per kg, preferably 1.0 to 250 mg per kg of mammal body weight, and are used in man in a unit dosage form, administered, one to four times daily, in the amount of 1 to 1,000 mg per unit dose.

For oral administration, fine powders or granules may contain diluting, dispersing and/or surface active agents, and may be presented in a draught, in water or in a syrup; in capsules or sachets in the dry state or in an aqueous or non-aqueous solution or suspension, wherein suspending agents may be included; in tablets, wherein binders and lubricants may be included; or in a suspension in water or a syrup. Where desirable or necessary, flavoring, preserving, suspending, thickening or emulsifying agents may be included. Tablets and granules are preferred, and these may be coated.

For parenteral administration or for administration as drops, as for eye infections, the compound may be presented in a solution or suspension in a concentration of from about 0.1 to 10%, more preferably 0.1 to 7%, most preferably 0.2% w/v. The solution may contain antioxidants, buffers, etc.

Alternatively, for infections of the eye, or other external tissues, e.g. mouth and skin, the compositions are preferably applied to the infected part of the body of the patient as a topical ointment or cream. The compounds may be presented in an ointment, for instance, with a water soluble ointment base, or in a cream, for instance with an oil in water cream base, in concentration of from about 0.1 to 10%, preferably 0.1 to 7%, most preferably 1% w/v.

The following non-limiting Example illustrates the preparation of a compound of the present invention. All temperatures are in °C.

## EXAMPLE 1

### 1-[(2-hydroxy-1,3,2-dioxophosphorinan-5-yl)oxymethyl]cytosine P-oxide.

To a stirred suspension of 1-[(1,3-dihydroxy-2-propoxy)methyl]cytosine (200 mg 0.93 mmol) in sieve-dried $CH_3CN$ (150 ml) was added $SNCL_4$ (0.15 mL, 1.29 mmol) and dissolution slowly occurred over 1 hour at room temperature. To this stirred solution was added dropwise (over 90 minutes) a solution of pyrophosphoryl chloride (0.4 mL, 2.7 mmol) in $CH_3CN$ (60 ml). After stirring for an additional 2 hours the mixture was neutralized to pH 6.4 by the addition of a saturated solution of $NaHCO_3$ and the mixture was stored in the refrigerator overnight. The solids were filtered off and the filtrate was evaporated to dryness in vacuo. This residue (1.3 g) was dissolved in $H_2O$ (4 mL) and the pH adjusted to 0.35 before being applied to a Darco (20-40 mesh) activated carbon column (12 g). The column was developed with $H_2O$ until no $CL^-$ was detected in the washings by the $AgNO_3$ test (~3L) and then with 5% $NH_4OH$ in 50% aqueous EtOH. Fractions containing the title compound were pooled and evaporated to dryness to give 190 mg of crude product as the $NH_4^+$ salts. This material was dissolved in $H_2O$ and applied to a Dowex 1 X 8 ($HCOO^-$form) column (200-400 mesh, 10 mL). The column was developed first with $H_2O$ and then with a linear gradient of $H_2O$ to 2N HCOOH. Fractions containing the required product were pooled and evaporated to dryness several times from $H_2O$, and their lyophilized to give 32 mg of the title compound as a white powder, which was a single peak upon evaluation by HPLC. 2H NMR ($D_2O$ $\delta$ from TSP): 3.87 (m, O-CH($CH_2O_2$, 4.17-4.49 (m, $CH_2OP$), 5.36 (S, N-$CH_2$-0), 6.09(d, J=8.0 Hz, $H_5$), 7.66 (d, J=8.0 Hz, $H_6$).

| | | | |
|---|---|---|---|
| Anal. Calc. for $C_8H_{12}N_3O_6$ P. 1.2 $H_2O$: | C, 32.16; | H, 4.86; | N, 14.06. |
| Found: | C, 31.97; | H, 4.55; | N, 13.81. |

## EXAMPLE 2

Activity of sodium 1-[(2-hydroxy-1,3,2-dioxophosphorinan-5-yl)-oxymethyl]cytosine P-oxide against human cytomegalovirus

Activity against Human Cytomegalovirus is determined by employing the Human Cytomegalovirus (HCMV) Assay. Two tissue culture assays are available; Cell Protection and Plaque Reduction. Both assays utilize MRC-5 cells (human fetal lung) and Eisenhart Human Cytomegalovirus. The following is a description of the Cell Protection and Plaque Reduction Assays.

Cell Protection Assay

Eight-two fold dilutions are made of the compound to be tested. After the dilutions are made, 0.5 ml of each dilution is added to each of three decanted wells of MRC-5. Two wells are used to determine the antiviral activity of the compound to be tested and one well is used to determine toxicity. The plates are then incubated at 37° C/5% $CO_2$ for one hour. After one hour 0.5 ml of Human Cytomegalovirus is added to the two wells used to test antiviral activity which contain the compound to be tested, and 0.5 ml of media is added to the one well used to determine toxicity. The plates are then placed back at 37° C/5% $CO_2$. After 5 days of incubation at 37° C/5% $CO_2$, the plates are read to determine antiviral activity and the degree of protection provided by the compound being tested.

Plaque Reduction Assay

Eight-two fold dilutions are made of the compound to be tested. After the dilutions are made, 0.5 ml of each dilution is added to each of three decanted wells of MRC-5. Two wells are used to determine the antiviral activity of the compound to be tested and one well is used to determine toxicity. The MRC-5 plates are then decanted, and the wells are infected with 0.1 ml of Human Cytomegalovirus inoculum containing ~60 PFUs. The plates are then adsorbed for 1 hour at 37° C/5% $CO_2$. After one hour the virus is removed from the wells and duplicate wells are refed with dilutions of the compound to be tested. The plates are then incubated at 37° C/5% $CO_2$. Plaques are counted microscopically on day 6 post inoculation.

The following chart shows the activity of the three compounds against Human Cytomegalovirus utilizing the above described Assays.

| | Cell Prot µg/ml | Plaq Red µg/ml |
|---|---|---|
| Sodium 1-[(2-hydroxy-1,3,2-dioxophosphorinan-5-yl)-oxymethyl]cytosine P-oxide | >25 | 25.00 |
| Sodium 9-[(2-hydroxy-1,3,2-dioxophosphorinan-5-yl)-oxymethyl]guanine P-oxide | 0.780 | 0.19 |
| 2-amino-1,9-dihydro-9-((2-hydroxy-1-(hydroxymethyl)-ethoxy)methyl)-6H-Purin-6-one | 1.560 | 0.78 |

## Claims

1. A compound having the formula:

B-$CH_2$-X-Z

wherein:

B is a pyrimidine, a substituted pyrimidine, a purine or substituted purine wherein the substituents on the purine or pyrimidine are selected from amino, hydroxyl, halogen, thio or alkylthiol wherein the alkyl moiety of the alkylthiol has 1 to 6 carbon atoms; and the purine or substituted purine is not guanine;

X is oxygen, sulfur or methylene;
Z is

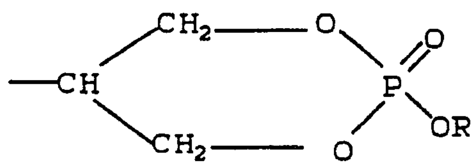

OR

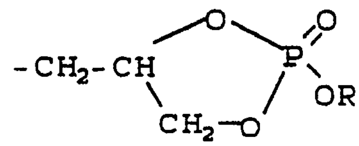

R is hydrogen or a pharmaceutically acceptable cation.

2. A compound according to Claim 1 wherein the purine or substituted purine is attached at its 9-position and the pyrimidine or substituted pyrimidine is attached at its 1-position.

3. A compound according to Claim 2 wherein:
B is adenine, thymine, cytosine or uracil;
X is oxygen, sulfur or methylene;
Z is

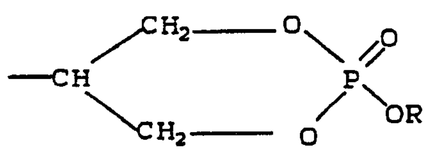

OR

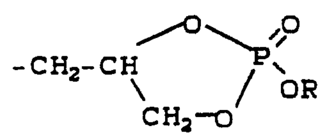

R is hydrogen or a pharmaceutically acceptable cation.

4. A compound according to Claim 3 wherein B is cytosine; X is oxygen; Z is;

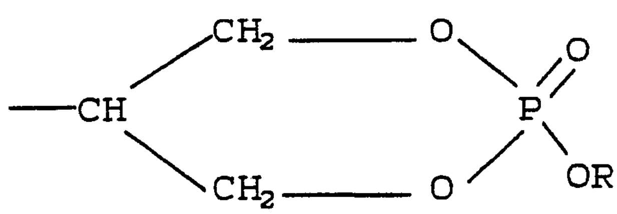

and R is hydrogen or a pharmaceutically acceptable cation.

5. 1-[(2-hydroxy-1,3,2-dioxophosphorinan-5-yl)-oxymethyl]cytosine P-oxide according to Claim 1.

6. An anti-viral pharmaceutical composition comprising an effective amount of a compound of Claim 1 and a pharmaceutically acceptable carrier.

7. The use of a compound of claim 1, for the preparation of a medicament useful for treating viral infections.